# EUROPEAN PATENT APPLICATION

(11) **EP 4 414 454 A1**
(43) Date of publication of application: **14.08.2024**
(21) Application number: 22878632.3
(22) Date of filing: 07.10.2022
(51) Int. Cl.: C12N 15/54, A23L 33/175, C12N 1/15, C12N 1/19, C12N 1/21, C12N 5/10, C12N 9/10, C12N 15/63

(54) **MODIFIED TRANSGLUTAMINASE**

(30) Priority: 07.10.2021 JP 2021165778
(71) Applicant: Amano Enzyme Inc., Nagoya-shi Aichi 460-8630 (JP)
(72) Inventor: YOSHIDA, Kazunori, Kakamigahara-shi, Gifu 509-0109 (JP); UEDA, Kenta, Kakamigahara-shi, Gifu 509-0109 (JP)
(74) Representative: Müller-Boré & Partner Patentanwälte PartG mbB
(86) International application number: PCT/JP2022/037716
(87) International publication number: WO 2023/058765

(57) **Abstract**

The purpose of the present invention is to provide a modified transglutaminase that has improved reactivity in a heating temperature range. A transglutaminase, which includes a polypeptide having an amino acid sequence derived from an amino acid sequence represented by SEQ ID NO: 1 by: substitution of the combination of the three amino acid residues at the 249 to 251 positions by a combination of three predetermined amino acid residues; substitution of the combination of the three amino acid residues at the 243 to 245 positions by a combination of three predetermined amino acid residues; and/or substitution of the amino acid residue at the 2 position by a predetermined amino acid, has improved reactivity in a heating temperature range compared to the polypeptide having the amino acid sequence represented by SEQ ID NO: 1.

## Description

### TECHNICAL FIELD

The present invention relates to a modified transglutaminase. More specifically, the present invention relates to a transglutaminase modified with a mutation that acquires high reactivity at any temperature of 50°C or higher.

### BACKGROUND ART

Transglutaminase is an enzyme that catalyzes an acyl transfer reaction of a γ-carboxylamide group of a glutamine residue in a polypeptide, and when an ε-amino group of a lysine residue is allowed to act as an acyl receptor, an ε-(γ-Gln)-Lys cross-linking is formed in or between molecules of a polypeptide chain.

That is, transglutaminase can modify a protein or a peptide by crosslinking. Specifically, a transglutaminase derived from the genus Streptomyces (see, for example, Patent Document 1) is used for meat biding, and production of sausages, bean curd, bread, and noodles. In addition, the use of transglutaminase has been studied not only in the food field but also in the fiber field, the medical field, the cosmetic field, and the like. Since such high usefulness is expected, attempts have been made to improve various properties (heat resistance, specific activity, substrate specificity, stability, and the like) of transglutaminase (see, for example, Patent Documents 2 to 4 and Non-Patent Documents 1 to 4).

### PRIOR ART DOCUMENTS

### PATENT DOCUMENTS

Patent Document 1: Japanese Patent Laid-open Publication No. H04-108381
Patent Document 2: Japanese Patent Laid-open Publication No. 2002-253272
Patent Document 3: Japanese Patent Laid-open Publication No. 2008-194004
Patent Document 4: WO 2019/107288

### NON-PATENT DOCUMENTS

Non-Patent Document 1: Marx CK et al., J. Biotechnol. 2008 Sep 10; 136(3-4): 156-162.
Non-Patent Document 2: Tagami U et al., Protein Eng. Des. Sel. 2009 Dec; 22(12): 747-752.
Non-Patent Document 3: Yokoyama K et al., Appl. Microbiol. Biotechnol. (2010) 87: 2087-2096.
Non-Patent Document 4: Buettner K et al., Amino Acids. 2012 Feb; 42(2-3): 987-996.

### SUMMARY OF THE INVENTION

### PROBLEMS TO BE SOLVED BY THE INVENTION

As described above, improvement of transglutaminase has been attempted so far for the purpose of improving various properties thereof, but in view of the possibility of further expanding the use expected from its high usefulness and the like, a new option of improved transglutaminase is desired. In particular, in industrial use of transglutaminase, heating conditions are often used, and high reactivity in a heating temperature range can be particularly useful properties.

Thus, the purpose of the present invention is to provide a modified transglutaminase with improved reactivity in a heating temperature range.

### MEANS FOR SOLVING THE PROBLEM

As a result of intensive studies, the present inventors have found as new mutations that provide transglutaminase with improved reactivity in a heating temperature range: (I) a substitution of a combination of 249th to 251st three amino acid residues in an amino acid sequence set forth in SEQ ID NO: 1 with a combination of predetermined three amino acid residues; (II) a substitution of a combination of 243rd to 245th three amino acid residues with a combination of predetermined three amino acid residues; and/or (III) a substitution of an amino acid residue at position 2 with a predetermined amino acid residue. The present invention has been completed based on this finding. That is, the present invention provides inventions of the following aspects.

Item 1. A modified transglutaminase including a polypeptide as defined in any of (I-1) to (I-3) below:
(I-1) a polypeptide including an amino acid sequence in which a combination of 249th to 251st three amino acid residues is substituted with a combination of three amino acid residues shown in any of (1) to (41) in Table 1 below in the amino acid sequence set forth in SEQ ID NO: 1,

**[Table 1]**

| | | | | | | | | | | | |
|---|---|---|---|---|---|---|---|---|---|---|---|
| (1) | RLF | (8) | ARF | (15) | LTF | (22) | VLF | (29) | CRY | (36) | LFY |
| (2) | RFY | (9) | CVF | (16) | LAF | (23) | VAF | (30) | CLW | (37) | RLW |
| (3) | GGF | (10) | DAF | (17) | MVF | (24) | VCF | (31) | CTY | (38) | RWY |
| (4) | LGF | (11) | GLF | (18) | RVF | (25) | VMF | (32) | FVY | (39) | SFY |
| (5) | VGF | (12) | ISF | (19) | SRF | (26) | VRF | (33) | GSY | (40) | TLY |
| (6) | RGY | (13) | LSF | (20) | TRF | (27) | VEF | (34) | LRW | (41) | VLY |
| (7) | VGL | (14) | LFF | (21) | TLF | (28) | YQF | (35) | LSY | | |

(I-2) a polypeptide in which, in an amino acid sequence in which a combination of 249th to 251st three amino acid residues in the amino acid sequence set forth in SEQ ID NO: 1 is substituted with a combination of three amino acid residues shown in any of (1) to (41) above, one or several amino acid residues other than the combination of three amino acid residues into which the substitution has been introduced are substituted, added, inserted or deleted, and transglutaminase activity at any temperature of 50°C or higher is improved more than transglutaminase activity at the temperature of a polypeptide including an amino acid sequence in which the combination of three amino acid residues is not substituted (that is, a reference polypeptide including the same amino acid sequence as the amino acid sequence obtained by the substitution, addition, insertion or deletion, except that the combination of 249th to 251st three amino acid residues is not substituted), and
(I-3) a polypeptide in which, in an amino acid sequence in which a combination of 249th to 251st three amino acid residues in the amino acid sequence set forth in SEQ ID NO: 1 is substituted with a combination of three amino acid residues shown in any of (1) to (41) above, sequence identity excluding the combination of three amino acid residues into which the substitution has been introduced with respect to the amino acid sequence set forth in SEQ ID NO: 1 is 70% or more, and transglutaminase activity at any temperature of 50°C or higher is improved more than transglutaminase activity at the temperature of a polypeptide including an amino acid sequence in which the combination of three amino acid residues is not substituted (that is, a reference polypeptide including the same amino acid sequence as the amino acid sequence having 70% or more sequence identity, except that the combination of 249th to 251st three amino acid residues is not substituted).

Item 2. A modified transglutaminase including a polypeptide as defined in any of (II-1) to (II-3) below:
(II-1) a polypeptide including an amino acid sequence in which a combination of 243rd to 245th three amino acid residues is substituted with a combination of three amino acid residues shown in any of (42) to (60) in Table 2 below in an amino acid sequence set forth in SEQ ID NO: 1,

**[Table 2]**

| | | | | | | | | | | | |
|---|---|---|---|---|---|---|---|---|---|---|---|
| (42) | MPT | (45) | SPS | (48) | RPR | (51) | CGR | (54) | KAL | (57) | MSA |
| (43) | RPT | (46) | LPS | (49) | VPL | (52) | CIR | (55) | LRS | (58) | MSL |
| (44) | SPR | (47) | LPR | (50) | ASS | (53) | ESR | (56) | LGS | (59) | MMR |
| | | | | | | | | | | (60) | QWS |

(II-2) a polypeptide in which, in an amino acid sequence in which a combination of 243rd to 245th three amino acid residues in the amino acid sequence set forth in SEQ ID NO: 1 is substituted with a combination of three amino acid residues shown in any of (42) to (60) above, one or several amino acid residues other than the combination of three amino acid residues into which the substitution has been introduced are substituted, added, inserted or deleted, and transglutaminase activity at any temperature of 50°C or higher is improved more than transglutaminase activity at the temperature of a polypeptide including an amino acid sequence in which the combination of three amino acid residues is not substituted (that is, a reference polypeptide including the same amino acid sequence as the amino acid sequence obtained by the substitution, addition, insertion or deletion, except that the combination of 243rd to 245th three amino acid residues is not substituted), and
(II-3) a polypeptide in which, in an amino acid sequence in which a combination of 243rd to 245th three amino acid residues in the amino acid sequence set forth in SEQ ID NO: 1 is substituted with a combination of three amino acid residues shown in any of (42) to (60) above, sequence identity excluding the combination of three amino acid residues into which the substitution has been introduced with respect to the amino acid sequence set forth in SEQ ID NO: 1 is 70% or more, and transglutaminase activity at any temperature of 50°C or higher is improved more than transglutaminase activity at the temperature of a polypeptide including an amino acid sequence in which the combination of three amino acid residues is not substituted (that is, a reference polypeptide including the same amino acid sequence as the amino acid sequence having 70% or more sequence identity, except that the combination of 243rd to 245th three amino acid residues is not substituted).

Item 3. A modified transglutaminase including a polypeptide as defined in any of (III-1) to (III-3) below:
(III-1) a polypeptide including an amino acid sequence in which a second amino acid residue is substituted with (61) L or (62) F in an amino acid sequence set forth in SEQ ID NO: 1,
(III-2) a polypeptide in which, in the amino acid sequence in which a second amino acid residue in the amino acid sequence set forth in SEQ ID NO: 1 is substituted with an amino acid residue (61) or (62), one or several amino acid residues other than the second amino acid residue are substituted, added, inserted or deleted, and transglutaminase activity at any temperature of 50°C or higher is improved more than transglutaminase activity at the temperature of a polypeptide including an amino acid sequence in which the second amino acid residue is not substituted (that is, a reference polypeptide including the same amino acid sequence as the amino acid sequence obtained by the substitution, addition, insertion or deletion, except that the second amino acid residue is not substituted), and
(III-3) a polypeptide in which, in the amino acid sequence in which a second amino acid residue in the amino acid sequence set forth in SEQ ID NO: 1 is substituted with an amino acid residue (61) or (62), sequence identity excluding the second amino acid residue with respect to the amino acid sequence set forth in SEQ ID NO: 1 is 70% or more, and transglutaminase activity at any temperature of 50°C or higher is improved more than transglutaminase activity at the temperature of a polypeptide including an amino acid sequence in which the second amino acid residue is not substituted (that is, a reference polypeptide including the same amino acid sequence as the amino acid sequence having 70% or more sequence identity, except that the second amino acid residues is not substituted).

Item 4. A DNA encoding the modified transglutaminase according to any one of items 1 to 3.

Item 5. An expression cassette or a recombinant vector including the DNA according to item 4.

Item 6. A transformant obtained by transforming a host with the expression cassette or recombinant vector according to item 5.

Item 7. A method for producing the modified transglutaminase according to any one of items 1 to 3, including a step of culturing the transformant according to item 6.

Item 8. An enzyme agent including the modified transglutaminase according to any one of items 1 to 3.

Item 9. A modifier of a protein material, including the modified transglutaminase according to any one of items 1 to 3.

Item 10. A method for producing a modified protein material, including a step of allowing the modified transglutaminase according to any one of items 1 to 3 to act on a protein material.

Item 11. The production method according to item 10, in which the protein material is edible.

### ADVANTAGES OF THE INVENTION

According to the present invention, a modified transglutaminase with improved reactivity in a heating temperature range is provided.

### BRIEF DESCRIPTION OF THE DRAWINGS

Fig. 1 shows results of a casein cross-linking reaction by a transglutaminase of Comparative Example 1 under each temperature condition.
Fig. 2 shows results of a casein cross-linking reaction by a transglutaminase of Comparative Example 2 under each temperature condition.
Fig. 3 shows results of a casein cross-linking reaction by a modified transglutaminase of Example 1 under each temperature condition.
Fig. 4 shows results of a casein cross-linking reaction by a modified transglutaminase of Example 2 under each temperature condition.
Fig. 5 shows results of a casein cross-linking reaction by a modified transglutaminase of Example 43 under each temperature condition.

### EMBODIMENTS OF THE INVENTION

Hereinafter, the present invention will be described in detail. Other than in the sequence listing, 20 types of amino acid residues in the amino acid sequence may be represented by one-letter abbreviations. That is, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P.

In the present specification, the amino acid sequence to be displayed is N-terminal at the left end and C-terminal at the right end.

As used herein, the term "non-polar amino acid" includes alanine, valine, leucine, isoleucine, proline, methionine, phenylalanine, and tryptophan. The term "uncharged amino acid" includes glycine, serine, threonine, cysteine, tyrosine, asparagine, and glutamine. The term "acidic amino acid" includes aspartic acid and glutamic acid. The term "basic amino acid" includes lysine, arginine, and histidine.

In the present specification, the term "substitution" includes not only a case where a substitution of an amino acid residue is artificially introduced, but also a case where a substitution of an amino acid residue is naturally introduced, that is, a case where amino acid residues are originally different. In the present specification, the substitution of the amino acid residue may be an artificial substitution or a natural substitution, but an artificial substitution is preferable.

### 1. Modified transglutaminase

The modified transglutaminase of the present invention is a polypeptide having (I) a substitution of a combination of 249th to 251st three amino acid residues in an amino acid sequence set forth in SEQ ID NO: 1 with a combination of predetermined three amino acid residues; (II) a substitution of a combination of 243rd to 245th three amino acid residues with a combination of predetermined three amino acid residues; and/or (III) a substitution of an amino acid residue at position 2 with a predetermined amino acid residue. Specifically, the modified transglutaminase of the present invention includes a polypeptide as defined in any of the following (I-1) to (I-3), any of the following (II-1) to (II-3), or any of the following (III-1) to (III-3).

(I-1) A polypeptide including an amino acid sequence in which a combination of 249th to 251st three amino acid residues is substituted with a combination of three amino acid residues shown in any of (1) to (41) in Table 3 below in the amino acid sequence set forth in SEQ ID NO: 1,

**[Table 3]**

| | | | | | | | | | | | |
|---|---|---|---|---|---|---|---|---|---|---|---|
| (1) | RLF | (8) | ARF | (15) | LTF | (22) | VLF | (29) | CRY | (36) | LFY |
| (2) | RFY | (9) | CVF | (16) | LAF | (23) | VAF | (30) | CLW | (37) | RLW |
| (3) | GGF | (10) | DAF | (17) | MVF | (24) | VCF | (31) | CTY | (38) | RWY |
| (4) | LGF | (11) | GLF | (18) | RVF | (25) | VMF | (32) | FVY | (39) | SFY |
| (5) | VGF | (12) | ISF | (19) | SRF | (26) | VRF | (33) | GSY | (40) | TLY |
| (6) | RGY | (13) | LSF | (20) | TRF | (27) | VEF | (34) | LRW | (41) | VLY |
| (7) | VGL | (14) | LFF | (21) | TLF | (28) | YQF | (35) | LSY | | |

(I-2) a polypeptide in which, in an amino acid sequence in which a combination of 249th to 251st three amino acid residues in the amino acid sequence set forth in SEQ ID NO: 1 is substituted with a combination of three amino acid residues shown in any of (1) to (41) above, one or several amino acid residues other than the combination of three amino acid residues into which the substitution has been introduced are substituted, added, inserted or deleted, and transglutaminase activity at any temperature of 50°C or higher is improved more than transglutaminase activity at the temperature of a polypeptide including an amino acid sequence in which the combination of three amino acid residues is not substituted (that is, a reference polypeptide including the same amino acid sequence as the amino acid sequence obtained by the substitution, addition, insertion or deletion, except that the combination of 249th to 251st three amino acid residues is not substituted), and
(I-3) a polypeptide in which, in an amino acid sequence in which a combination of 249th to 251st three amino acid residues in the amino acid sequence set forth in SEQ ID NO: 1 is substituted with a combination of three amino acid residues shown in any of (1) to (41) above, sequence identity excluding the combination of three amino acid residues into which the substitution has been introduced with respect to the amino acid sequence set forth in SEQ ID NO: 1 is 70% or more, and transglutaminase activity at any temperature of 50°C or higher is improved more than transglutaminase activity at the temperature of a polypeptide including an amino acid sequence in which the combination of three amino acid residues is not substituted (that is, a reference polypeptide including the same amino acid sequence as the amino acid sequence having 70% or more sequence identity, except that the combination of 249th to 251st three amino acid residues is not substituted).

(II-1) A polypeptide including an amino acid sequence in which a combination of 243rd to 245th three amino acid residues is substituted with a combination of three amino acid residues shown in any of (42) to (60) in Table 4 below in an amino acid sequence set forth in SEQ ID NO: 1,

**[Table 4]**

| | | | | | | | | | | | |
|---|---|---|---|---|---|---|---|---|---|---|---|
| (42) | MPT | (45) | SPS | (48) | RPR | (51) | CGR | (54) | KAL | (57) | MSA |
| (43) | RPT | (46) | LPS | (49) | VPL | (52) | CIR | (55) | LRS | (58) | MSL |
| (44) | SPR | (47) | LPR | (50) | ASS | (53) | ESR | (56) | LGS | (59) | MMR |
| | | | | | | | | | | (60) | QWS |

(II-2) a polypeptide in which, in an amino acid sequence in which a combination of 243rd to 245th three amino acid residues in the amino acid sequence set forth in SEQ ID NO: 1 is substituted with a combination of three amino acid residues shown in any of (42) to (60) above, one or several amino acid residues other than the combination of three amino acid residues into which the substitution has been introduced are substituted, added, inserted or deleted, and transglutaminase activity at any temperature of 50°C or higher is improved more than transglutaminase activity at the temperature of a polypeptide including an amino acid sequence in which the combination of three amino acid residues is not substituted (that is, a reference polypeptide including the same amino acid sequence as the amino acid sequence obtained by the substitution, addition, insertion or deletion, except that the combination of 243rd to 245th three amino acid residues is not substituted), and
(II-3) a polypeptide in which, in an amino acid sequence in which a combination of 243rd to 245th three amino acid residues in the amino acid sequence set forth in SEQ ID NO: 1 is substituted with a combination of three amino acid residues shown in any of (42) to (60) above, sequence identity excluding the combination of three amino acid residues into which the substitution has been introduced with respect to the amino acid sequence set forth in SEQ ID NO: 1 is 70% or more, and transglutaminase activity at any temperature of 50°C or higher is improved more than transglutaminase activity at the temperature of a polypeptide including an amino acid sequence in which the combination of three amino acid residues is not substituted (that is, a reference polypeptide including the same amino acid sequence as the amino acid sequence having 70% or more sequence identity, except that the combination of 243rd to 245th three amino acid residues is not substituted).

(III-1) A polypeptide including an amino acid sequence in which a second amino acid residue is substituted with (61) L or (62) F in an amino acid sequence set forth in SEQ ID NO: 1,
(III-2) a polypeptide in which, in the amino acid sequence in which a second amino acid residue in the amino acid sequence set forth in SEQ ID NO: 1 is substituted with an amino acid residue (61) or (62), one or several amino acid residues other than the second amino acid residue are substituted, added, inserted or deleted, and transglutaminase activity at any temperature of 50°C or higher is improved more than transglutaminase activity at the temperature of a polypeptide including an amino acid sequence in which the second amino acid residue is not substituted (that is, a reference polypeptide including the same amino acid sequence as the amino acid sequence obtained by the substitution, addition, insertion or deletion, except that the second amino acid residue is not substituted), and
(III-3) a polypeptide in which, in the amino acid sequence in which a second amino acid residue in the amino acid sequence set forth in SEQ ID NO: 1 is substituted with an amino acid residue (61) or (62), sequence identity excluding the second amino acid residue with respect to the amino acid sequence set forth in SEQ ID NO: 1 is 70% or more, and transglutaminase activity at any temperature of 50°C or higher is improved more than transglutaminase activity at the temperature of a polypeptide including an amino acid sequence in which the second amino acid residue is not substituted (that is, a reference polypeptide including the same amino acid sequence as the amino acid sequence having 70% or more sequence identity, except that the second amino acid residues is not substituted).

The polypeptide including the amino acid sequence set forth in SEQ ID NO: 1 is itself a mutant of wild-type transglutaminase (mature form) derived from Streptomyces mobaraensis. Specifically, the polypeptide including the amino acid sequence set forth in SEQ ID NO: 1 is a mutant having five-fold mutation (S2P/S23Y/Y24N/H289Y/K294L), in which an amino acid residue at position 2 (S), an amino acid residue at position 23 (S), an amino acid residue at position 24 (Y), an amino acid residue at position 289 (H), and an amino acid residue at position 294 (K) of the wild-type transglutaminase (mature form) derived from Streptomyces mobaraensis including an amino acid sequence set forth in SEQ ID NO: 2 are substituted with P, Y, N, Y, and L, respectively.

The polypeptides shown in (I-1) to (I-3), (II-1) to (II-3), and (III-1) to (III-3) include not only polypeptides obtained by artificial substitution but also polypeptides originally having such amino acid sequences.

In the polypeptides of (I-2), (II-2), and (III-2), the amino acid modification to be introduced may include only one modification from among substitution, addition, insertion, and deletion (for example, only substitution), or may include two or more modifications (for example, substitution and insertion). In the polypeptides of (I-2), (II-2), and (III-2), the number of amino acid differences at any difference site may be 1 or several, and is, for example, 1 to 90, preferably 1 to 80, 1 to 70, 1 to 60, 1 to 50, 1 to 40, or 1 to 30, more preferably 1 to 20, 1 to 10, 1 to 8, 1 to 7, 1 to 6, 1 to 5, or 1 to 4, further preferably 1 to 3, and particularly preferably 1 or 2, or 1.

Also, in the polypeptides of (I-3), (II-3), and (III-3), the sequence identity to the amino acid sequence set forth in SEQ ID NO: 1 may be 70% or more, but is preferably 75% or more, 79% or more, 85% or more, more preferably 90% or more, further preferably 95% or more, still more preferably 98% or more, even still more preferably 98.5% or more, particularly preferably 99% or more, 99.5% or more, or 99.9% or more.

Here, in the polypeptides of (I-3), (II-3), and (III-3), the sequence identity to each amino acid sequence set forth in SEQ ID NO: 1 is a sequence identity calculated by comparison with the amino acid sequence set forth in SEQ ID NO: 1. Further, the "sequence identity" refers to a value of amino acid sequence identity obtained by bl2seq program (Tatiana A. Tatsusova, Thomas L. Madden, FEMS Microbiol. Lett., Vol. 174, p247-250, 1999) in BLASTPACKAGE [sgi32bit edition, Version 2.0.12; available from National Center for Biotechnology Information (NCBI)]. Parameters may be set to Gap insertion Cost value:11 and Gap extension Cost value:1.

In the polypeptides of (I-2), (II-2), (III-2), (I-3), (11-3), and (III-3), from the viewpoint of further improving reactivity in a heating temperature range, it is more preferable that no substitution or deletion has been introduced into an amino acid residue at position 23 (Y), an amino acid residue at position 24 (N), and an amino acid residue at position 294 (L) in the amino acid sequence set forth in SEQ ID NO: 1. In addition, since an amino acid residue at position 64 (C) is a transglutaminase active center, it is preferable not to introduce a substitution or deletion into this site. Furthermore, since an amino acid residue at position 64 (C), an amino acid residue at position 255 (D), and an amino acid residue at position 274 (H), including the transglutaminase active center, correspond to the catalytic triad residues, it is also preferable not to introduce substitutions or deletions into these sites.

When an amino acid substitution has been introduced into SEQ ID NO: 1 in the polypeptides of (I-2), (II-2), (III-2), (I-3), (II-3), and (III-3), a conservative substitution is a preferred aspect of the introduced amino acid substitution. That is, examples of the substitution in the polypeptides of (I-2), (II-2), (III-2), (I-3), (II-3), and (III-3) include: when the amino acid before substitution is a non-polar amino acid a substitution with another non-polar amino acid; when the amino acid before substitution is a non-charged amino acid, a substitution with another non-charged amino acid; when the amino acid before substitution is an acidic amino acid, a substitution with another acidic amino acid; and when the amino acid before substitution is a basic amino acid, a substitution with another basic amino acid.

The polypeptides shown in (I-1) to (I-3), (II-1) to (II-3), and (III-1) to (III-3) have transglutaminase activity, and have improved reactivity in a heating temperature range. In the present invention, the heating temperature range refers to any temperature of 50°C or higher, preferably 55°C or higher, more preferably 60°C or higher, further preferably 65°C or higher, and still more preferably 70°C or higher. The upper limit of the heating temperature range is not particularly limited, and is, for example, 90°C or lower, and preferably 85°C or lower.

In addition, in the polypeptides of (I-2), (II-2), (I-3), and (II-3), the phrase "transglutaminase activity at any temperature of 50°C or higher is improved more than transglutaminase activity at the temperature of a polypeptide including an amino acid sequence in which the combination of three amino acid residues is not substituted" means that the transglutaminase activity at any predetermined temperature selected from a heating temperature range of 50°C or higher is only required to be improved more than the transglutaminase activity at the predetermined temperature of the reference polypeptide including an amino acid sequence in which a combination of the three amino acid residues is not substituted. The degree of improvement is not particularly limited, but is preferably 1.1 times or more, 1.2 times or more, more preferably 1.3 times or more, 1.4 times or more, further preferably 1.5 times or more, 1.6 times or more, still more preferably 1.7 times or more, 1.8 times or more, 1.9 times or more, even still more preferably 2 times or more, 2.1 times or more, 2.2 times or more, 2.3 times or more, 2.4 times or more, particularly preferably 2.5 times or more, 2.6 times or more, 2.7 times or more, or 2.8 times or more the transglutaminase activity of the reference polypeptide at the predetermined temperature. The upper limit of these ranges is not particularly limited, and is, for example, 5 times or less, 4 times or less, or 3.5 times or less.

In the polypeptides of (III-2) and (III-3), the phrase "transglutaminase activity at any temperature of 50°C or higher is improved more than transglutaminase activity at the temperature of a polypeptide including an amino acid sequence in which the second amino acid residue is not substituted" means that the transglutaminase activity at any predetermined temperature selected from a heating temperature range of 60°C or higher is improved more than the transglutaminase activity at the predetermined temperature of the reference polypeptide including an amino acid sequence in which the second amino acid residue is not substituted, and as the degree of improvement, the degree of improvement in the transglutaminase activity at any predetermined temperature selected from a heating temperature range of 60°C to 65°C is 1.3 times or more, preferably 1.4 times or more, more preferably 1.5 times or more, further preferably 1.6 times or more the transglutaminase activity at the predetermined temperature of the reference polypeptide. The upper limit of these ranges is not particularly limited, and is, for example, 5 times or less, 4 times or less, or 3.5 times or less.

In the transglutaminase activity, an enzyme activity of producing 1 µmol of hydroxamic acid per minute using Z-Gln-Gly (benzyloxycarbonyl-L-glutaminylglycine) and hydroxylammonium chloride as substrates is defined as 1 unit (1 U).

Preferred examples of the polypeptides of (I-2), (II-2), (III-2), (I-3), (II-3), and (III-3) shown above include a polypeptide including a combination of three amino acid residues shown in at least any of the above (1) to (60) or an amino acid sequence substituted with an amino acid residue shown in the above (61) or (62) in the following predetermined transglutaminase including an amino acid sequence having high sequence identity to the amino acid sequence of SEQ ID NO: 1. Examples of the predetermined transglutaminase include a transglutaminase derived from Streptomyces mobaraensis and including an amino acid sequence set forth in SEQ ID NO: 2, a transglutaminase derived from Streptomyces albireticuli and including an amino acid sequence set forth in NCBI Reference Sequence: WP_087929495.1 (sequence identity to SEQ ID NO: 1 is 82.4%), a transglutaminase derived from Streptomyces luteireticuli and including an amino acid sequence shown in GenBank: AAR31178.1 (sequence identity to SEQ ID NO: 1 is 82.0%), a transglutaminase derived from Streptomyces cinnamoneus and including an amino acid sequence set forth in GenBank: CAA70055.1 (sequence identity to SEQ ID NO: 1 is 79.6%), a transglutaminase derived from Streptomyces platensis and including an amino acid sequence set forth in GenBank: AAS84612.1 (sequence identity to SEQ ID NO: 1 is 79.9%), a transglutaminase derived from Streptomyces hygroscopicus and including an amino acid sequence set forth in GenBank: ATI36569.1 (sequence identity to SEQ ID NO: 1 is 79.9%), and the like.

### 2. DNA

The DNA of the present invention is a DNA encoding the modified transglutaminase described in "1. Modified transglutaminase" above.

The DNA of the present invention is not particularly limited as long as it is a DNA having a base sequence encoding a modified transglutaminase including the polypeptides defined in (I-1) to (I-3), (II-1) to (II-3), and (III-1) to (III-3) described in "1. Modified transglutaminase" above. Examples of the base sequence of the DNA encoding the amino acid sequence set forth in SEQ ID NO: 1, which is a reference sequence of the polypeptides shown in (I-1) to (I-3), (II-1) to (II-3), and (III-1) to (III-3), include SEQ ID NO: 3 (base sequence of DNA encoding five-fold mutant (S2P/S23Y/Y24N/H289Y/K294L) of transglutaminase derived from Streptomyces mobaraensis). Therefore, the DNA of the present invention can be appropriately designed by those skilled in the art using SEQ ID NO: 3 as a reference sequence.

Examples of the DNA of the present invention include DNA shown in any of the following items [i] to [iii].

[i] A DNA including a base sequence in which positions 745 to 753 of a base sequence set forth in SEQ ID NO: 3 are substituted with any of the base sequences [1] to [41] shown in Table 5 below, positions 727 to 735 of the base sequence set forth in SEQ ID NO: 3 are substituted with any of the base sequences [42] to [60] shown in Table 5 below, and/or positions 4 to 6 of the base sequence set forth in SEQ ID NO: 3 are substituted with any of the base sequences [61] to [62] shown in Table 5 below.

A DNA including a base sequence in which positions 745 to 753 of the base sequence set forth in SEQ ID NO: 3 are substituted with any of the base sequences [1] to [41] shown in Table 5 below encodes a polypeptide including an amino acid sequence in which a combination of 249th to 251st three amino acid residues is substituted with a combination of three amino acid residues shown in any of (1) to (41) in Tables 1 and 3 above (the polypeptide shown in (I-1) above), respectively, in the amino acid sequence set forth in SEQ ID NO: 1. A DNA including a base sequence in which positions 727 to 735 of the base sequence set forth in SEQ ID NO: 3 are substituted with any of the base sequences [42] to [60] shown in Table 5 below encodes a polypeptide including an amino acid sequence in which a combination of 243rd to 245th three amino acid residues is substituted with a combination of three amino acid residues shown in any of (42) to (60) in Tables 2 and 4 above (the polypeptide shown in (II-1) above), respectively, in the amino acid sequence set forth in SEQ ID NO: 1. A DNA including a base sequence in which positions 4 to 6 of the base sequence set forth in SEQ ID NO: 3 are substituted with any of the base sequences [61] to [62] shown in Table 5 below encodes a polypeptide including an amino acid sequence in which a second amino acid residue is substituted with an amino acid residue shown in any of (61) to (62) above (the polypeptide shown in (III-1) above) in the amino acid sequence set forth in SEQ ID NO: 1.

**[Table 5]**

| | Substitution to positions 745 to 753 of SEQ ID NO: 3 | | |
|---|---|---|---|
| [1] | CGN, AGR | CTN, TTR | TTY |
| [2] | CGN, AGR | TTY | TAY |
| [3] | GGN | GGN | TTY |
| [4] | CTN, TTR | GGN | TTY |
| [5] | GTN | GGN | TTY |
| [6] | CGN, AGR | GGN | TAY |
| [7] | GTN | GGN | CTN, TTR |
| [8] | GCN | CGN, AGR | TTY |
| [9] | TGY | GTN | TTY |
| [10] | GAY | GCN | TTY |
| [11] | GGN | CTN, TTR | TTY |
| [12] | ATH | TCN, AGY | TTY |
| [13] | CTN, TTR | TCN, AGY | TTY |
| [14] | CTN, TTR | TTY | TTY |
| [15] | CTN, TTR | ACN | TTY |
| [16] | CTN, TTR | GCN | TTY |
| [17] | ATG | GTN | TTY |
| [18] | CGN, AGR | GTN | TTY |
| [19] | TCN, AGY | CGN, AGR | TTY |
| [20] | ACN | CGN, AGR | TTY |
| [21] | ACN | CTN, TTR | TTY |
| [22] | GTN | CTN, TTR | TTY |
| [23] | GTN | GCN | TTY |
| [24] | GTN | TGY | TTY |
| [25] | GTN | ATG | TTY |
| [26] | GTN | CGN, AGR | TTY |
| [27] | GTN | GAR | TTY |
| [28] | TAY | CAR | TTY |
| [29] | TGY | CGN, AGR | TAY |
| [30] | TGY | CTN, TTR | TGG |
| [31] | TGY | ACN | TAY |
| [32] | TTY | GTN | TAY |
| [33] | GGN | TCN, AGY | TAY |
| [34] | CTN, TTR | CGN, AGR | TGG |
| [35] | CTN, TTR | TCN, AGY | TAY |
| [36] | CTN, TTR | TTY | TAY |
| [37] | CGN, AGR | CTN, TTR | TGG |
| [38] | CGN, AGR | TGG | TAY |
| [39] | TCN, AGY | TTY | TAY |
| [40] | ACN | CTN, TTR | TAY |
| [41] | GTN | CTN, TTR | TAY |

| | Substitution to positions 727 to 735 of SEQ ID NO: 3 | | |
|---|---|---|---|
| [42] | ATG | CCN | ACN |
| [43] | CGN, AGR | CCN | ACN |
| [44] | TCN, AGY | CCN | CGN, AGR |
| [45] | TCN, AGY | CCN | TCN, AGY |
| [46] | CTN, TTR | CCN | TCN, AGY |
| [47] | CTN, TTR | CCN | CGN, AGR |
| [48] | CGN, AGR | CCN | CGN, AGR |
| [49] | GTN | CCN | CTN, TTR |
| [50] | GCN | TCN, AGY | TCN, AGY |
| [51] | TGY | GGN | CGN, AGR |
| [52] | TGY | ATH | CGN, AGR |
| [53] | GAR | TCN, AGY | CGN, AGR |
| [54] | AAR | GCN | CTN, TTR |
| [55] | CTN, TTR | CGN, AGR | TCN, AGY |
| [56] | CTN, TTR | GGN | TCN, AGY |
| [57] | ATG | TCN, AGY | GCN |
| [58] | ATG | TCN, AGY | CTN, TTR |
| [59] | ATG | ATG | CGN, AGR |
| [60] | CAR | TGG | TCN, AGY |

| | Substitution to positions 4 to 6 of SEQ ID NO: 3 | | |
|---|---|---|---|
| [61] | CTN, TTR | | |
| [62] | TTY | | |

[ii] A DNA encoding a polypeptide in which the transglutaminase activity at any temperature of 50°C or higher is improved more than the transglutaminase activity at the temperature of the polypeptide including the amino acid sequence set forth in SEQ ID NO: 1, the DNA hybridizing under stringent conditions with a DNA including a base sequence complementary to the DNA set forth in the above [i].

Here, the phrase "stringent conditions" refers to conditions of incubating in 6 × SSC (1 × SSC is 0.15 M NaCl, 0.015 M sodium citrate, pH 7.0) containing 0.5% SDS, 5 × Denhartz's [0.1% bovine serum albumin (BSA), 0.1% polyvinylpyrrolidone, 0.1% Ficoll 400] and 100 µg/ml salmon sperm DNA for 4 hours to overnight at 50°C to 65°C.

Hybridization under stringent conditions is specifically performed by the following method. That is, a nylon membrane on which a DNA library or a cDNA library is immobilized is prepared, and the nylon membrane is blocked at 65°C in a prehybridization solution containing 6 × SSC, 0.5% SDS, 5 × Denhartz's, and 100 µg/ml salmon sperm DNA. Each probe labeled ³²P is then added and incubated overnight at 65°C. This nylon membrane is washed in 6 × SSC, at room temperature for 10 minutes, in 2 × SSC containing 0.1% SDS, at room temperature for 10 minutes, in 0.2 × SSC containing 0.1% SDS, at 45°C for 30 minutes, and then autoradiography is performed, and DNA specifically hybridized with the probe can be detected.

[iii] A DNA encoding a polypeptide in which the transglutaminase activity at any temperature of 50°C or higher is improved more than the transglutaminase activity at the temperature of the polypeptide including the amino acid sequence set forth in SEQ ID NO: 1 and having 70% or more homology to the DNA set forth in the above [i]. The homology is preferably 75% or more, 79% or more, 85% or more, more preferably 90% or more, further preferably 95% or more, still more preferably 98% or more, even still more preferably 98.5% or more, particularly preferably 99% or more, 99.5% or more, or 99.9% or more.

Here, the "homology" of DNA is calculated using publicly or commercially available software with an algorithm to compare a reference sequence as a query sequence. Specifically, BLAST, FASTA, GENETYX (manufactured by GENETYX CORPORATION), or the like can be used, and these may be used by being set as default parameters.

The DNA of the present invention can be obtained, for example, by introducing a combination of three amino acid residues shown in any of the above (1) to (60) or a mutation that substitutes for an amino acid residue shown in the above (61) or (62) into a DNA encoding a transglutaminase including the amino acid sequence set forth in SEQ ID NO: 1. The DNA of the present invention can also be artificially synthesized by a total gene synthesis method.

When a DNA including a base sequence set forth in SEQ ID NO: 3 is used as the DNA encoding the polypeptide including the amino acid sequence set forth in SEQ ID NO: 1, the DNA including a base sequence set forth in SEQ ID NO: 3 can be isolated from genomic DNA of a five-fold mutant (S2P/S23Y/Y24N/H289Y/K294L) of transglutaminase derived from Streptomyces mobaraensis by a conventional method using PCR.

A method for introducing a specific mutation into a specific site of a base sequence is known, and for example, a site-specific mutation introduction method of DNA or the like can be used. Examples of a specific method for converting a base in DNA include use of a commercially available kit (QuickChange Lightning Site-Directed Mutagenesis kit: Agilent Technology, KOD-Plus-Mutagenesis kit: manufactured by Toyobo Co., Ltd., and the like) and the like.

The base sequence of DNA into which a mutation has been introduced can be confirmed using a DNA sequencer. Once the base sequence is determined, the DNA encoding the polypeptide can be obtained by chemical synthesis, PCR using a cloned probe as a template, or hybridization using a DNA fragment having the base sequence as a probe.

In addition, it is possible to synthesize a mutant form of the DNA encoding the polypeptide and having a function equivalent to that before mutation by a site-directed mutagenesis method or the like. A mutation can be introduced into the DNA encoding a polypeptide by a known method such as a Kunkel method, a Gapped duplex method, or a megaprimer PCR method.

The DNA of the present invention is preferably one in which the codon usage frequency is optimized for a host, and more preferably DNA in which the codon usage frequency is optimized for E. coli.

As an index representing the codon usage frequency, the total host optimal codon usage frequency of each codon may be adopted. The optimal codon is defined as a codon having the highest usage frequency among codons corresponding to the same amino acid. The codon usage frequency is not particularly limited as long as it is optimized for the host, and examples of the optimal codon of E. coli include the following. F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serine (agc), P: proline (ccg), T: threonine (acc), A: alanine (gcg), C: cysteine (tgc), W: tryptophan (tgg), R: arginine (cgc), G: glycine (ggc).

### 3. Expression cassette or recombinant vector

The expression cassette or recombinant vector of the present invention contains the DNA described in "2. DNA" above (hereinafter, it is described as "the DNA of the present invention".). The expression cassette or recombinant vector of the present invention can be obtained by linking a promoter and a terminator to the DNA of the present invention, or by inserting the expression cassette of the present invention or the DNA of the present invention into an expression vector.

The expression cassette of the present invention or the recombinant vector of the present invention may further contain, as a control factor, a transcription element such as an enhancer, a CCAAT box, a TATA box, or an SPI site, as necessary, in addition to a promoter and a terminator. These control factors may be operably linked to the DNA of the present invention. Operably linked means that various control factors that regulate the DNA of the present invention and the DNA of the present invention are linked in a state where they can operate in a host cell.

As for the recombinant vector of the present invention, an expression vector constructed for genetic recombination from a phage, a plasmid, or a virus capable of autonomously replicating in a host is suitable. Such expression vectors are known, and examples of commercially available expression vectors include pQE vectors (QIAGEN K.K.), pDR540, pRIT2T (GE Healthcare Biosciences), pET vectors (Merck Corporation), and the like. The expression vector may be used by selecting an appropriate combination with a host cell. For example, when E. coli is used as a host cell, a combination of a pET vector and a DH5α E. coli strain, a combination of a pET vector and a BL21(DE3) E. coli strain, a combination of a pDR540 vector and a JM109 E. coli strain, or the like may be used.

### 4. Transformant

The transformant of the present invention is obtained by transforming a host with the expression cassette or recombinant vector described in "3. Expression cassette or recombinant vector" above.

The host used for the production of the transformant is not particularly limited as long as it is one to which a gene can be introduced, the expression cassette or the recombinant vector is stable, it is capable of autonomous replication, and it can express the trait of a gene containing the DNA of the present invention. For example, suitable examples thereof include bacteria belonging to the genus Escherichia such as Escherichia coli, the genus Bacillus such as Bacillus subtilis, the genus Pseudomonas such as Pseudomonas putida, and the like; yeast, and the like, but the host may also be an animal cell, an insect cell, a plant, or the like.

The transformant of the present invention can be obtained by introducing the expression cassette of the present invention or the recombinant vector of the present invention into a host. The site into which the DNA of the present invention is introduced is not particularly limited as long as a target gene can be expressed, and may be on a plasmid or on a genome. Specific examples of a method for introducing the expression cassette of the present invention or the recombinant vector of the present invention include a recombinant vector method and a genome editing method. Conditions for introducing the expression cassette or the recombinant vector into a host may be appropriately set according to the type of the host and the like. When the host is a bacterium, examples thereof include a method using a competent cell by calcium ion treatment, an electroporation method, and the like. When the host is a yeast, examples thereof include an electroporation method, a spheroplast method, a lithium acetate method, and the like. When the host is an animal cell, examples thereof include an electroporation method, a calcium phosphate method, a lipofection method, and the like. When the host is an insect cell, examples thereof include a calcium phosphate method, a lipofection method, an electroporation method, and the like. When the host is a plant cell, examples thereof include an electroporation method, an Agrobacterium method, a particle gun method, a PEG method, and the like.

Whether or not the expression cassette of the present invention or the recombinant vector of the present invention has been incorporated into a host can be confirmed by a PCR method, a Southern hybridization method, a Northern hybridization method, and the like.

When it is confirmed whether or not the expression cassette of the present invention or the recombinant vector of the present invention has been incorporated into a host by a PCR method, for example, genomic DNA or the expression cassette or the recombinant vector may be separated and purified from a transformant.

The separation and purification of the expression cassette or the recombinant vector are performed based on a lysate obtained by lysing bacteria, for example, when the host is a bacterium. As a lysis method, for example, treatment is performed with a lytic enzyme such as lysozyme, and as necessary, a protease, other enzymes, and a surfactant such as sodium lauryl sulfate (SDS) are used in combination.

Furthermore, physical crushing methods such as freeze thawing and French press processing may be combined. The separation and purification of a DNA from a lysate can be performed, for example, by appropriately combining a deproteinization treatment using a phenol treatment and a protease treatment, a ribonuclease treatment, an alcohol precipitation treatment, and a commercially available kit.

DNA cleavage can be performed according to a conventional method, for example, using a restriction enzyme treatment. As a restriction enzyme, for example, a type II restriction enzyme that acts on a specific nucleotide sequence is used. Binding between the DNA and the expression cassette or the expression vector is performed, for example, using a DNA ligase.

Thereafter, PCR is performed by designing a primer specific to the DNA of the present invention using the separated and purified DNA as a template. The amplification product obtained by PCR is subjected to agarose gel electrophoresis, polyacrylamide gel electrophoresis, capillary electrophoresis, or the like, and is stained with ethidium bromide, SYBR Green solution, and the like, and then the amplification product is detected as a band, so that transformation can be confirmed.

In addition, PCR can be performed using a primer labeled with a fluorescent dye or the like in advance to detect an amplification product. Furthermore, a method of binding an amplification product to a solid phase such as a microplate and confirming the amplification product by fluorescence, an enzymatic reaction, or the like may also be adopted.

### 5. Method for producing modified transglutaminase

The method for producing a modified transglutaminase of the present invention is a method for producing the enzyme according to "1. Modified transglutaminase" above, and includes a step of culturing the transformant of the present invention. When a predetermined mutation shown in any of (1) to (62) contained in the modified transglutaminase has been naturally introduced, the modified transglutaminase can be obtained by a production method including a step of culturing a microorganism that produces the modified transglutaminase.

The culture conditions may be appropriately set in consideration of the nutritional physiological properties of the transformant or the microorganism, and liquid culture is preferably mentioned. Also, in the case of performing industrial production, ventilating/stirring culture is preferred.

As a nutrient source of the medium, those required for growth of the transformant or the microorganism can be used. A carbon source may be any assimilable carbon compound, and examples thereof include glucose, sucrose, lactose, maltose, molasses, pyruvic acid, and the like.

A nitrogen source may be any assimilable nitrogen compound, and examples thereof include peptone, meat extract, yeast extract, casein hydrolysate, soybean cake alkaline extract, and the like.

In addition to the carbon source and the nitrogen source, for example, phosphates, carbonates, sulfates, salts of magnesium, calcium, potassium, iron, manganese, zinc, and the like, specific amino acids, specific vitamins, and the like may be used as necessary.

The culture temperature can be appropriately set in a range in which the transformant or the microorganism of the present invention can grow and the transformant or the microorganism produces the modified transglutaminase, but is preferably about 15 to 37°C. The culture may be completed at an appropriate time with reference to the time when the modified transglutaminase reaches the highest yield, and the culture time is usually about 12 to 48 hours.

After the transformant or the microorganism is cultured, the culture solution is collected by a method such as centrifugation to recover a supernatant or bacterial cells, the bacterial cells are treated by a mechanical method such as ultrasonic wave and French press or a lytic enzyme such as lysozyme, and as necessary, are solubilized by using an enzyme such as protease or a surfactant such as sodium lauryl sulfate (SDS), whereby a water-soluble fraction containing the modified transglutaminase can be obtained.

In addition, by selecting an appropriate expression cassette or expression vector and a host, the expressed modified transglutaminase can also be secreted into the culture solution.

The water-soluble fraction containing the modified transglutaminase obtained as described above may be subjected to a purification treatment as it is, or may be subjected to a purification treatment after the modified transglutaminase in the water-soluble fraction is concentrated.

The concentration can be performed by, for example, concentration under reduced pressure, membrane concentration, a salting-out treatment, fractionation precipitation with a hydrophilic organic solvent (for example, methanol, ethanol, and acetone), or the like.

The purification treatment of the modified transglutaminase can be performed, for example, by appropriately combining methods such as gel filtration, adsorption chromatography, ion exchange chromatography, and affinity chromatography.

The modified transglutaminase thus purified can be pulverized by freeze drying, vacuum drying, spray drying, or the like, as necessary, and then distributed to the market.

### 6. Enzyme agent

The modified transglutaminase can be provided in a form of an enzyme agent. Therefore, the present invention also provides an enzyme agent containing the modified transglutaminase described in "1. Modified transglutaminase" above as an active ingredient.

The content of the modified transglutaminase in the enzyme agent of the present invention is not particularly limited, and is, for example, 1 U/g or more, preferably 10 U/g or more, more preferably 100 U/g or more, further preferably 250 U/g or more, and particularly preferably 500 U/g or more, 1000 U/g or more, or 2000 U/g or more.

The enzyme agent of the present invention may contain other components to the extent that the effect of the present invention is not affected, in addition to the modified transglutaminase. Examples of the other components include enzymes other than the modified transglutaminase, additives, culture residues generated by the production method, and the like.

Examples thereof include amylase (α-amylase, β-amylase, glucoamylase), glucosidase (α-glucosidase, β-glucosidase), galactosidase (α-galactosidase, β-galactosidase), protease (acidic protease, neutral protease, alkaline protease), peptidase (leucine peptidase, aminopeptidase), lipase, esterase, cellulase, phosphatase (acidic phosphatase, alkaline phosphatase), nuclease, deaminase, oxidase, dehydrogenase, glutaminase, pectinase, catalase, dextranase, protein deamidase, pullulanase, and the like. These other enzymes may be contained singly or in combination of a plurality of kinds thereof.

Examples of the additives include an excipient, a buffer agent, a suspension agent, a stabilizer, a preservative, an antiseptic, physiological saline, and the like. Examples of the excipient include starch, dextrin, maltose, trehalose, lactose, D-glucose, sorbitol, D-mannitol, sucrose, glycerol, and the like. Examples of the buffer agent include phosphate, citrate, acetate, and the like. Examples of the stabilizer include propylene glycol, ascorbic acid, and the like. Examples of the preservative include phenol, benzalkonium chloride, benzyl alcohol, chlorobutanol, methylparaben, and the like. Examples of the antiseptic include ethanol, benzalkonium chloride, paraoxybenzoic acid, chlorobutanol, and the like. These additives may be contained singly or in combination of a plurality of kinds thereof.

Examples of the culture residues include components derived from a culture medium, contaminant proteins, microbial cell components, and the like.

The form of the enzyme agent of the present invention is not particularly limited, and examples thereof include a liquid form, a solid form (such as powder or granules), and the like. The composition can be prepared by a generally known method.

### 7. Modifier of protein material

The modified transglutaminase can be used for a known use of transglutaminase. For example, a modified transglutaminase can be used for the purpose of modifying a protein material. Accordingly, the present invention also provides a modifier of a protein material containing a modified transglutaminase.

A specific aspect of the modification of the protein material is not particularly limited, and may be a property change caused by ε-(γ-Gln)-Lys cross-linking of the protein molecule. Specifically, examples of the modification of the protein material include adhesion of the protein material, and the like. A specific method of using the modifier of the protein material is as described in "8. Method for producing modified protein material" below.

### 8. Method for producing modified protein material

As described above, the modified transglutaminase can be used for the purpose of modifying a protein material. Therefore, the present invention also provides a method for producing a modified protein material, including a step of allowing the modified transglutaminase to act on a protein material.

In the production method of the present invention, a mixture containing a protein material and a modified transglutaminase is subjected to action conditions of the modified transglutaminase to allow a reaction for modifying a protein to proceed.

The protein material is not particularly limited as long as it is a material containing a protein, and may be either edible or non-edible. The edible protein material can be used as food and drink or as a material for producing food and drink. The non-edible protein material can be used as a material for protein experiment, a medical material, a fiber material, a cosmetic material, or the like.

Specific examples of the protein material include a protein source itself and a preparation obtained by performing a treatment for increasing the protein content from the protein source by a known method, and these are appropriately selected by those skilled in the art. Examples of the edible protein material include a preparation obtained from a food product containing a plant protein as a plant protein material; and a food product containing an animal protein or a preparation prepared therefrom as an animal protein material. Examples of the edible plant protein include bean proteins such as soybean proteins, broad bean proteins, pea proteins, chickpea proteins, mung bean proteins, and lupin bean proteins; grain proteins such as wheat protein, rye protein, oat protein, and corn protein; and the like. Examples of the edible animal protein include milk, fish and shellfish meat, livestock meat, tendons, and the like. Examples of the non-edible protein material include casein, albumin, and globulin derived from biological samples such as milk, egg white, and serum; and silk, wool, and the like.

Among the action conditions of the modified transglutaminase, the temperature condition is not particularly limited, but since the transglutaminase activity in the heating temperature range of the modified transglutaminase is improved, the temperature condition is preferably a heating condition. Specific temperature conditions include, for example, 50°C or higher, preferably 60°C or higher, more preferably 65°C or higher, further preferably 68°C or higher, still more preferably 74°C or higher, and even still more preferably 78°C or higher. The upper limit of the heating condition is not particularly limited, and is, for example, 90°C or lower, 85°C or lower, or 82°C or lower.

After completion of the reaction, an enzyme deactivation treatment is performed, followed by cooling and, as necessary, post-treatment, whereby a modified protein material is obtained.

### EXAMPLES

Hereinafter, the present invention will be specifically described with reference to examples, but the present invention is not construed as being limited to the following examples.

### Test Example 1

Modified transglutaminases (Examples 1 to 60), which are mutants of a transglutaminase which is a polypeptide including the amino acid sequence set forth in SEQ ID NO: 1 (Comparative Example 1), were prepared by the following method.

### 1. Preparation of mutant strain

### 1-1. Introduction of mutation

[1] PCR primers for predetermined mutation introduction of (1) to (60) shown in Table 1 (Table 3) and Table 2 (Table 4) were appropriately designed.
[2] PCR using plasmid pET20b into which a gene encoding transglutaminase of Comparative Example 1 (SEQ ID NO: 3) was incorporated as a template was performed using each PCR primer set (specifically, after a reaction at 98°C for 1 minute was performed, a reaction at 98°C for 10 seconds, at 60°C for 15 seconds, and at 68°C for 2 minutes was performed for 15 cycles, and the resultant was reacted at 68°C for 5 minutes and allowed to stand at 4°C.).
[3] DpnI (1.5 µL/tube) was added to the resulting PCR reaction solution (25 µL/tube) for treatment (37°C, 3 hours or more).
[4] Ligation treatment (16°C, overnight) was performed using T4 kinase.
[5] E. coli BL21 (DE3) was transformed with the resulting ligation reaction solution (11 µL/tube) and cultured (37°C, overnight) in an ampicillin-containing LB medium to obtain a strain into which the mutation was introduced.

### 1-2. Acquisition of enzyme extract

[1] The mutant strain was inoculated into an ampicillin-containing TB medium and cultured at 33°C for 48 hours. IPTG (final concentration 0.1 mM) was added at 24 hours from the start of culture.
[2] The culture solution was centrifuged (3,000 g × 10 minutes), and then the supernatant was removed to recover bacterial cells.
[3] A bacteriolytic agent was added to lyse the bacterial cells.
[4] The lysate was centrifuged (3,000 g × 10 minutes), and then the supernatant was recovered as an enzyme extract.

### 1-3. Maturation (removal of propeptide sequence)

[1] The enzyme extract and 2 mg/mL protease (dispase) solution were mixed in equal amounts, and the mixture was reacted (30°C, 2 hours or more).
[2] The mixed liquid was centrifuged (3,000 g × 10 minutes), and then the supernatant was recovered to obtain a matured enzyme (modified transglutaminase). [0095]

### 2. Purification of modified transglutaminase

Each modified transglutaminase was purified according to the attached protocol using TALON Spin columns (Takara Bio Inc.) and HisTALON Buffer Set (Takara Bio Inc.). The purified modified transglutaminase was used for characterization of a mutant enzyme.

### 3. Characterization of modified transglutaminase

2.42 g of 2-amino-2-hydroxymethyl-1.3-propanediol, 0.70 g of hydroxyammonium hydrochloride, 0.31 g of reduced glutathione and 1.01 g of Z-Gln-Gly (benzyloxycarbonyl-L-glutaminylglycine) were dissolved in distilled water to make a total amount of 100 mL (pH 6.0), thereby obtaining a substrate solution (R-1). On the other hand, 30 mL of a 3 M hydrochloric acid solution, 30 mL of a 12% trichloroacetic acid solution and 30 mL of a 5% iron (III) chloride solution were mixed to obtain a coloring solution (R-2).

Each modified transglutaminase (matured enzyme) prepared was diluted to an appropriate concentration with 200 mM Tris-HCl (pH 6.0) to obtain a sample solution. 100 µL of the substrate solution (R-1) was added to and mixed with 10 µL of the sample solution, and then the mixture was reacted at 37°C for 10 minutes. 100 µL of the coloring solution (R-2) was added to stop the reaction and form an Fe complex, then the absorbance at 525 nm was measured. As a control, the absorbance of a solution reacted in the same manner using an enzyme liquid previously heat-inactivated was measured, and the difference in absorbance from the sample solution was determined. Separately, a calibration curve was prepared using L-glutamic acid-γ-monohydroxamic acid instead of the enzyme liquid, and the amount of hydroxamic acid produced was determined from the absorbance difference. The transglutaminase activity producing 1 µmol of hydroxamic acid per minute was defined as 1 unit (1 U).

### 3-1. Evaluation of reactivity under heating temperature conditions

Each modified transglutaminase was diluted 5 times with 200 mM Tris-HCl (pH 6.0) (sample solution), and transglutaminase activity was measured at a reaction temperature of 50°C, 60°C, 65°C, or 70°C.

Relative transglutaminase activity (%) of each modified transglutaminase when the transglutaminase activity at a reaction temperature of 60°C in Comparative Example 1 was 100%, was derived. The results are shown in Tables 6 and 7. As Comparative Example 2, a similar operation was performed using a transglutaminase derived from Streptomyces mobaraensis (wild-type).

**[Table 6]**

| | | Mutations introduced into SEQ ID NO: 1 E249Xa/G250Xb/F251Xc | | | Relative value (%) when specific activity at 60°C of Comparative Example 1 (reference polypeptide) is 100% | | | |
|---|---|---|---|---|---|---|---|---|
| | | Xa | Xb | Xc | 50°C | 60°C | 65°C | 170°C |
| Comparative Example 1 | *1 | E | G | F | 81% | 100% | 35% | 8% |
| **Example 1** | (1) | **R** | **L** | F | **218%** | **281%** | **71%** | **23%** |
| **Example 2** | (2) | **R** | **F** | **Y** | 51% | 75% | **58%** | 6% |
| **Example 3** | (3) | **G** | G | F | **98%** | **108%** | 23% | 8% |
| **Example 4** | (4) | **L** | G | F | 61% | 80% | **52%** | 4% |
| **Example 5** | (5) | **V** | G | F | 39% | 49% | **56%** | 4% |
| **Example 6** | (6) | **R** | G | **Y** | 51% | 87% | **104%** | 8% |
| **Example 7** | (7) | **V** | G | **L** | 56% | 67% | **90%** | 6% |
| **Example 8** | (8) | **A** | **R** | F | 65% | 93% | **136%** | 8% |
| **Example 9** | (9) | **C** | **V** | F | 56% | 67% | **81%** | 5% |
| **Example 10** | (10) | **D** | **A** | F | 55% | 69% | **71%** | 4% |
| **Example 11** | (11) | **G** | **L** | F | 73% | 74% | **51%** | 3% |
| **Example 12** | (12) | **I** | **S** | F | 47% | 69% | **85%** | 5% |
| **Example 13** | (13) | **L** | **S** | F | **92%** | **116%** | **52%** | **11%** |
| **Example 14** | (14) | **L** | **F** | F | **114%** | **113%** | 28% | 8% |
| **Example 15** | (15) | **L** | **T** | F | 77% | 93% | **111%** | 6% |
| **Example 16** | (16) | **L** | **A** | F | 69% | 81% | **107%** | 6% |
| **Example 17** | (17) | **M** | **V** | F | 69% | 84% | **97%** | 7% |
| **Example 18** | (18) | **R** | **V** | F | 58% | 77% | **102%** | 7% |
| **Example 19** | (19) | **S** | **R** | F | **115%** | **147%** | **59%** | **13%** |
| **Example 20** | (20) | **T** | **R** | F | **155%** | **173%** | **99%** | **22%** |
| **Example 21** | (21) | **T** | **L** | F | 78% | **107%** | **83%** | 6% |
| **Example 22** | (22) | **V** | **L** | F | **131%** | **147%** | **41%** | **13%** |
| **Example 23** | (23) | **V** | **A** | F | **108%** | **134%** | **64%** | **14%** |
| **Example 24** | (24) | **V** | **C** | F | **102%** | **116%** | 35% | **12%** |
| **Example 25** | (25) | **V** | **M** | F | 87% | **113%** | **39%** | **9%** |
| **Example 26** | (26) | **V** | **R** | F | 69% | **103%** | **68%** | **10%** |
| **Example 27** | (27) | **V** | **E** | F | 58% | 71% | **80%** | **10%** |
| **Example 28** | (28) | **Y** | Q | F | **108%** | **129%** | **103%** | 8% |
| **Example 29** | (29) | **C** | **R** | **Y** | **96%** | **121%** | **74%** | **16%** |
| **Example 30** | (30) | **C** | **L** | **W** | **84%** | 78% | **50%** | 4% |
| **Example 31** | (31) | **C** | **T** | **Y** | 50% | 71% | **87%** | 6% |
| **Example 32** | (32) | **F** | **V** | **Y** | **93%** | **106%** | 35% | **11%** |
| **Example 33** | (33) | **G** | **S** | **Y** | **95%** | **111%** | **51%** | **10%** |
| **Example 34** | (34) | **L** | **R** | **W** | **163%** | **211%** | **87%** | **15%** |
| **Example 35** | (35) | **L** | **S** | **Y** | **101%** | **116%** | **55%** | **13%** |
| **Example 36** | (36) | **L** | **F** | **Y** | 65% | 77% | **92%** | 4% |
| Example **37** | (37) | **R** | **L** | **W** | **161%** | **169%** | **37%** | 8% |
| **Example 38** | (38) | **R** | **W** | **Y** | 73% | **103%** | 33% | 8% |
| **Example 39** | (39) | **S** | **F** | **Y** | **127%** | **107%** | 32% | **10%** |
| **Example 40** | (40) | **T** | **L** | **Y** | 80% | 96% | **106%** | 6% |
| **Example 41** | (41) | **V** | **L** | **Y** | **91%** | **113%** | 34% | **10%** |
| Comparative Example 2 | *1 | E | G | F | 62% | 14% | 12% | 2% |

| | | | | | | | | |
|---|---|---|---|---|---|---|---|---|
| * 1: In Comparative Examples 1 and 2, no mutations (1) to (41) were introduced into positions 249 to 251 of SEQ ID NO: 1. Comparative Example 1: S2P/S23Y/Y24N/H289Y/K294L five-fold mutant of SEQ ID NO: 2 (SEQ ID NO: 1) Comparative Example 2: *Streptomyces mobaraensis* wild-type transglutaminase (SEQ ID NO: 2) | | | | | | | | |

**[Table 7]**

| | Mutations introduced into SEQ ID NO: 1 S243Xd/P244Xe/T245Xf | | | | Relative value (%) when specific activity at 60°C of Comparative Example 1 (reference polypeptide) is 100% | | | |
|---|---|---|---|---|---|---|---|---|
| | | Xd | Xe | Xf | 50°C | 60°C | 65°C | 70°C |
| Comparative Example 1 | *2 | S | P | T | 79% | 100% | 36% | 8% |
| **Example 42** | (42) | **M** | P | T | **104%** | **117%** | **40%** | **10%** |
| **Example 43** | (43) | **R** | P | T | **87%** | **106%** | 24% | 7% |
| **Example 44** | (44) | S | P | **R** | **119%** | **147%** | **49%** | **11%** |
| **Example 45** | (45) | S | P | **S** | **106%** | **120%** | 31% | 7% |
| **Example 46** | (46) | **L** | P | **S** | **97%** | **117%** | **43%** | **11%** |
| **Example 47** | (47) | **L** | P | **R** | **109%** | **113%** | 33% | **9%** |
| **Example 48** | (48) | **R** | P | **R** | **119%** | **123%** | 23% | 6% |
| **Example 49** | (49) | **V** | P | **L** | **90%** | **106%** | 30% | 7% |
| **Example 50** | (50) | **A** | **S** | **S** | **128%** | **139%** | **42%** | **12%** |
| **Example 51** | (51) | **C** | **G** | **R** | **158%** | **163%** | **39%** | **11%** |
| **Example 52** | (52) | **C** | I | **R** | **139%** | **147%** | **46%** | **11%** |
| **Example 53** | (53) | **E** | **S** | **R** | **88%** | **109%** | 32% | 8% |
| **Example 54** | (54) | **K** | **A** | **L** | **140%** | **145%** | 31% | **10%** |
| **Example 55** | (55) | **L** | **R** | **S** | **120%** | **120%** | 29% | **9%** |
| **Example 56** | (56) | **L** | **G** | **S** | **114%** | **114%** | **39%** | 6% |
| **Example 57** | (57) | **M** | **S** | **A** | **125%** | **134%** | 35% | **10%** |
| **Example 58** | (58) | **M** | **S** | **L** | **83%** | 100% | 34% | **10%** |
| **Example 59** | (59) | **M** | **M** | **R** | **118%** | **120%** | 26% | 8% |
| **Example 60** | (60) | **Q** | **W** | **S** | **107%** | **126%** | **38%** | **10%** |
| Comparative Example 2 | *2 | S | P | T | 66% | 16% | 6% | 5% |

| | | | | | | | | |
|---|---|---|---|---|---|---|---|---|
| *2: In Comparative Examples 1 and 2, no mutations (42) to (60) were introduced into positions 243 to 245 of SEQ ID NO: 1. Comparative Example 1: S2P/S23Y/Y24N/H289Y/K294L five-fold mutant of SEQ ID NO: 2 (SEQ ID NO: 1) Comparative Example 2: *Streptomyces mobaraensis* wild-type transglutaminase (SEQ ID NO: 2) | | | | | | | | |

As shown in Tables 6 and 7, all of the modified transglutaminases having a predetermined mutation of (1) to (60) had improved transglutaminase activity at at least any temperature of 50°C or higher as compared with the transglutaminase of Comparative Example 1.

### Test Example 2

Cross-linking activities of the transglutaminase of Comparative Examples and the modified transglutaminase of Examples shown in Table 8 and Table 9 under heating temperature conditions were examined by the method shown below.

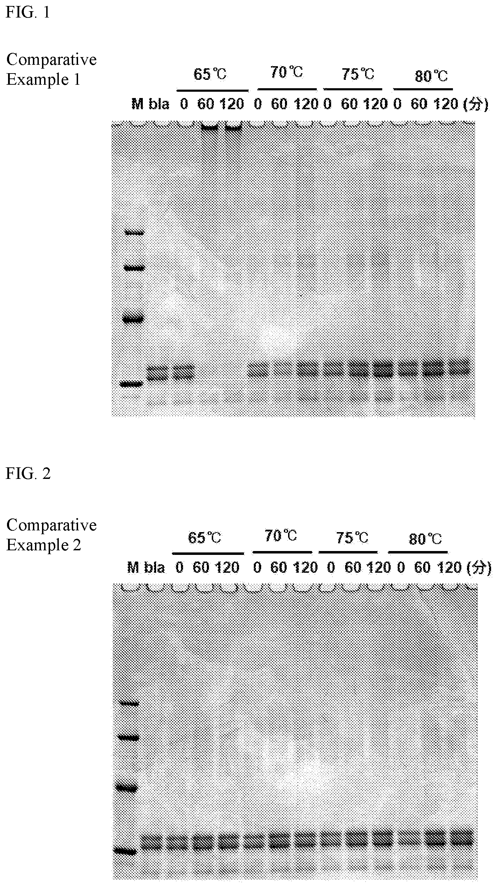

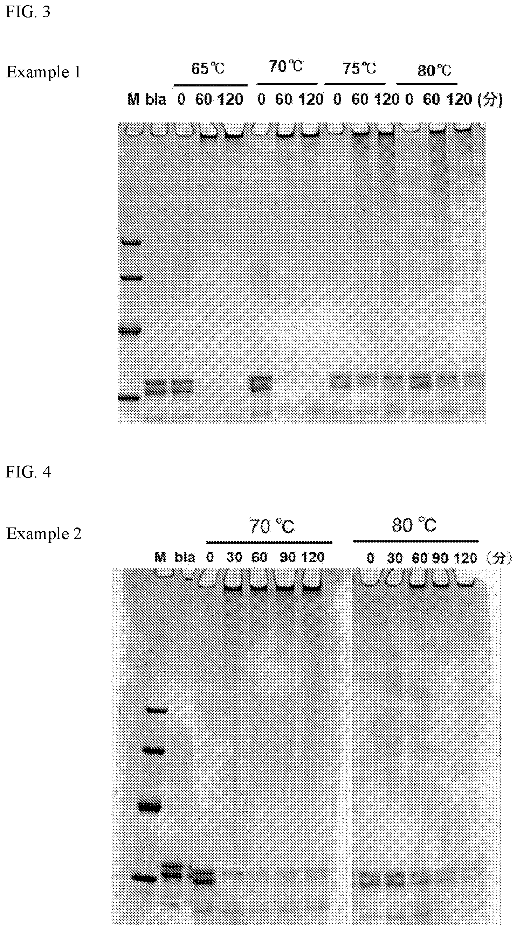

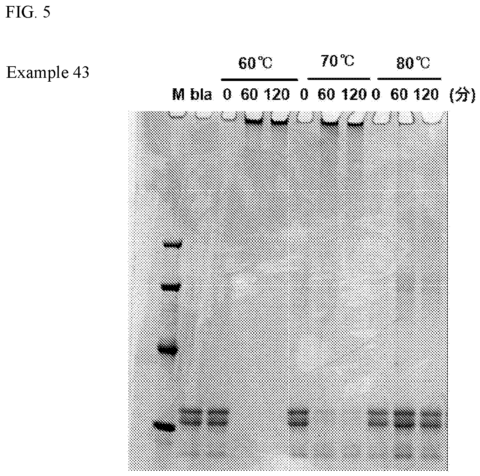

A reaction mixture containing 1% by weight of casein (substrate) and 20 µg of the modified transglutaminase in water was reacted at 60°C, 65°C, 70°C, 75°C or 80°C for 2 hours. The reaction mixture after the reaction was mixed with an SDS-PAGE sample buffer and boiled, then ice-cooled, and the cross-linking activity was evaluated by SDS-PAGE. The results at 60°C, 70°C, and 80°C are shown in Tables 8 and 9. In addition, for Comparative Examples and some Examples (Examples 1, 2, and 43), the results of examining the cross-linking activities during the reaction over time by SDS-PAGE are shown in Figs. 1 to 5. In Figs. 1 to 5, "bla" shows the result of reaction with only casein as a substrate (no enzyme), and "M" shows electrophoresis markers (Protein Molecular Weight Markers LMW, Global Life Science Technologies Japan K.K. Code No. 17044601).

**[Table 8]**

| | Mutations introduced into SEQ ID NO: 1 E249Xa/G250Xb/F251Xc | | | | Casein-cross-linking activity | | |
|---|---|---|---|---|---|---|---|
| | | Xa | Xb | Xc | 60°C | 70°C | 80°C |
| Comparative Example 1 | / | E | G | F | ○ | × | × |
| **Example 1** | (1) | **R** | **L** | F | ○ | ○ | ○ |
| **Example 2** | (2) | **R** | **F** | **Y** | ○ | ○ | ○ |
| **Example 16** | (16) | **L** | **A** | F | ○ | ○ | × |
| **Example 19** | (19) | **S** | **R** | F | ○ | ○ | × |
| **Example 20** | (20) | **T** | **R** | F | ○ | ○ | × |
| **Example 22** | (22) | **V** | **L** | F | ○ | ○ | × |
| **Example 23** | (23) | **V** | **A** | F | ○ | ○ | × |
| **Example 25** | (25) | **V** | **M** | F | ○ | ○ | × |
| **Example 26** | (26) | **V** | **R** | F | ○ | ○ | × |
| **Example 32** | (32) | **F** | **V** | **Y** | ○ | ○ | × |
| **Example 33** | (33) | **G** | **S** | **Y** | ○ | ○ | × |
| **Example 34** | (34) | **L** | **R** | **W** | ○ | ○ | × |
| **Example 35** | (35) | **L** | **S** | **Y** | ○ | ○ | × |
| **Example 36** | (36) | **L** | **F** | **Y** | ○ | ○ | × |
| **Example 37** | (37) | **R** | **L** | **W** | ○ | ○ | × |
| **Example 38** | (38) | **R** | **W** | **Y** | ○ | ○ | × |
| **Example 39** | (39) | **S** | **F** | **Y** | ○ | ○ | × |
| **Example 40** | (40) | **T** | **L** | **Y** | ○ | ○ | × |
| **Example 41** | (41) | **V** | **L** | **Y** | ○ | ○ | × |
| Comparative Example 2 | / | E | G | F | ○ | × | × |

**[Table 9]**

| | Mutations introduced into SEQ ID NO: 1 S243Xd/P244Xe/T245Xf | | | | Casein-cross-linking activity | | |
|---|---|---|---|---|---|---|---|
| | | Xd | Xe | Xf | 60°C | 70°C | 80°C |
| Comparative Example 1 | / | S | P | T | ○ | × | × |
| **Example 42** | (42) | **M** | P | T | ○ | ○ | × |
| **Example 43** | (43) | **R** | P | T | ○ | ○ | × |
| **Example 44** | (44) | S | P | **R** | ○ | ○ | × |
| **Example 46** | (46) | **L** | P | **S** | ○ | ○ | × |
| **Example 47** | (47) | **L** | P | **R** | ○ | ○ | × |
| **Example 48** | (48) | **R** | P | **R** | ○ | ○ | × |
| **Example 50** | (50) | **A** | **S** | **S** | ○ | ○ | × |
| **Example 52** | (52) | **C** | **I** | **R** | ○ | ○ | × |
| **Example 54** | (54) | **K** | **A** | **L** | ○ | ○ | × |
| **Example 57** | (57) | **M** | **S** | **A** | ○ | ○ | × |
| Comparative Example 2 | / | S | P | T | ○ | × | × |

As shown in Tables 8 and 9 and Figs. 1 to 5, the modified transglutaminases of Examples having a predetermined mutation enabled casein cross-linking at 70°C or higher, which could not be achieved by the transglutaminases of Comparative Examples. In particular, according to the modified transglutaminases of Example 1 and Example 2, excellent cross-linking activities were exhibited even at a high temperature of 80°C.

### Test Example 3

Modified transglutaminases (Examples 61 and 62), which are mutants of a transglutaminase which is a polypeptide including an amino acid sequence set forth in SEQ ID NO: 2 (Comparative Example 2), were prepared. Specifically, a modified transglutaminase was prepared in the same manner as in Examples 1 and 2 of Test Example 1 except that the reference sequence was changed to SEQ ID NO: 2, and the reactivity under heating temperature conditions was evaluated. The results are shown in Table 10. Table 10 also shows the results of Comparative Example 1 and Examples 1 and 2 in Test Example 1.

**[Table 10]**

| | Amino acids at positions 249 to 251 in SEQ ID NO: 1 E249Xa/G250Xb/F251Xc | | | | Relative value (%) when specific activity at 60°C of reference polypeptide is 100% | | |
|---|---|---|---|---|---|---|---|
| | | Xa | Xb | Xc | 60°C | 65°C | 70°C |
| Comparative Example 2 | *1 | E | G | F | 100% | 35% | 15% |
| **Example 61** | (1) | **R** | **L** | F | **111%** | **45%** | **18%** |
| **Example 62** | (2) | **R** | **F** | **Y** | 92% | **42%** | **17%** |
| Comparative Example 1 | *1 | E | G | F | 100% | 35% | **8%** |
| **Example 1** | (1) | **R** | **L** | F | **281%** | **71%** | **23%** |
| **Example 2** | (2) | **R** | **F** | **Y** | 75% | **58%** | 6% |

| | | | | | | | |
|---|---|---|---|---|---|---|---|
| *1: In Comparative Examples 2 and 1, no mutations (1) to (41) were introduced into positions 249 to 251 of SEQ ID NO: 1. Comparative Example 2 (reference polypeptides of Examples 61 and 62): *Streptomyces mobaraensis* wild-type transglutaminase (SEQ ID NO: 2) Comparative Example 1 (reference polypeptides of Examples 1 and 2): S2P/S23Y/Y24N/H289Y/K294L five-fold mutant of SEQ ID NO: 2 (SEQ ID NO: 1) | | | | | | | |

As shown in Table 10, in the modified transglutaminase having a predetermined mutation of (1) and (2), even when the portion other than the predetermined mutation was the same as that in the wild type (Examples 61 and 62), the transglutaminase activity was improved at least at any temperature of 50°C or higher as compared with the transglutaminase having no predetermined mutation (Comparative Example 2 and Comparative Example 1, respectively), as in the case where another mutation was further introduced (Examples 1 and 2).

### Test Example 4

Mutants in which the amino acid residue at position 2 of the transglutaminase which is a polypeptide including an amino acid sequence set forth in SEQ ID NO: 2 (Comparative Example 2) was substituted (Comparative Examples 3 and 4 and Examples 63 and 64) were prepared. Specifically, a modified transglutaminase was prepared in the same manner as in Test Example 1 except that the reference sequence was changed to SEQ ID NO: 2 and the substitution position was changed to position 2. Among them, the amino acid residue at position 2 of Comparative Example 3 was substituted with P which is the same as the polypeptide including the amino acid sequence set forth in SEQ ID NO: 1. Also, the amino acid residues at position 2 of Comparative Example 4 and Examples 63 and 64 were substituted with Y, L, and F, respectively. Thereby, Comparative Examples 2 and 4 and Examples 63 and 64 were prepared as modified transglutaminases in which the amino acid residue at position 2 of the transglutaminase of Comparative Example 3 (transglutaminase including an amino acid sequence having high sequence identity to the amino acid sequence of SEQ ID NO: 1) was substituted with S, Y, L, and F, respectively. Reactivity of these modified transglutaminases under heating temperature conditions was evaluated in the same manner as in Test Example 1. The results are shown in Table 11.

**[Table 11]**

| | Amino acids at each position in SEQ ID NO: 1 | | | | | Relative value (%) when specific activity at 60°C of Comparative Example 3 (reference polypeptide) is 100% | | |
|---|---|---|---|---|---|---|---|---|
| | **2** | 23 | 24 | 289 | 294 | 60°C | 65°C | 70°C |
| Comparative Example 3 | P | S | Y | H | K | 100% | 33% | 14% |
| Comparative Example 2 | S | S | Y | H | K | 37% | 14% | 8% |
| Comparative Example 4 | Y | S | Y | H | K | 117% | 37% | 13% |
| **Example 63** | **L** | S | Y | H | K | **142%** | **56%** | **15%** |
| **Example 64** | **F** | S | Y | H | K | **146%** | **55%** | 14% |

| | | | | | | | | |
|---|---|---|---|---|---|---|---|---|
| Comparative Example 3 (reference polypeptides of Examples 63 and 64 and Comparative Examples 2 and 4): S2P mutant of SEQ ID NO: 2 Comparative Example 2: *Streptomyces mobaraensis* wild-type transglutaminase (SEQ ID NO: 2) | | | | | | | | |

As shown in Table 11, in the modified transglutaminase having a predetermined (L or F) mutation at position 2 (Examples 63 and 64), the transglutaminase activity was improved at least at any temperature of 60°C or higher, and the transglutaminase activity improving effect at 60 to 65°C was observed to a remarkable extent of more than 1.4 times at each stage, as compared with the transglutaminase having no predetermined mutation (Comparative Example 3). Among the modified transglutaminases of Comparative Examples 2 and 3 having a mutation different from the predetermined mutation, the activity of transglutaminase was rather decreased in Comparative Example 2, and the activity of transglutaminase was hardly improved except that the specific activity at 60°C of Comparative Example 4 was slightly improved. That is, it was found that the remarkable transglutaminase activity-improving effect observed in Examples 63 and 64 is a specific effect obtained by having the predetermined mutation at position 2.

### Test Example 5

Modified transglutaminases, which are mutants in which positions 2, 101, 157, 208, 249, 250, 251, 275, and 284, or even position 289 of the transglutaminase which is a polypeptide including the amino acid sequence set forth in SEQ ID NO: 1 (Comparative Example 1) were substituted (Examples 65 and 66), were prepared. Specifically, modified transglutaminases were prepared in the same manner as in Example 2 of Test Example 1 except that substitutions at positions 2, 101, 157, 208, 275, 284, or even 289 were introduced in addition to the substitutions at positions 249, 250, and 251, and the reactivity under heating temperature conditions was evaluated. The results are shown in Table 12.

**[Table 12]**

| | Amino acids at each position in SEQ ID NO: 1 | | | | | | | | | | | | | Relative value (%) when specific activity at 60°C of polypeptide of Comparative Example 1 is 100% | | | |
|---|---|---|---|---|---|---|---|---|---|---|---|---|---|---|---|---|---|
| | **2** | 23 | 24 | 101 | 157 | 208 | **249** | **250** | **251** | 275 | 284 | 289 | 294 | 50°C | 60°C | 65°C | 70°C |
| Comparative Example 1 | P | Y | N | S | G | R | E | G | F | G | S | Y | L | 79% | 100% | 36% | 8% |
| **Example 65** | **L** | Y | N | P | R | E | **R** | **F** | **Y** | A | L | Y | L | 54% | 107% | 127% | 151% |
| **Example 66** | **L** | Y | N | P | R | E | **R** | **F** | **Y** | A | L | H | L | 97% | 189% | 216% | 263% |

| | | | | | | | | | | | | | | | | | |
|---|---|---|---|---|---|---|---|---|---|---|---|---|---|---|---|---|---|
| Comparative Example 1: S2P/S23Y/Y24N/H289Y/K294L five-fold mutant of SEQ ID NO: 2 (SEQ ID NO: 1) | | | | | | | | | | | | | | | | | |

As shown in Table 12, in the modified transglutaminases having the predetermined mutations at positions 2 and 249 to 251 (Examples 65 and 66), the transglutaminase activity was improved at least at any temperature of 50°C or higher, particularly at 60 to 70°C or 65 to 70°C, as compared with the transglutaminase having no predetermined mutation (Comparative Example 1).

## Claims

1. A modified transglutaminase comprising a polypeptide as defined in any of (I-1) to (I-3) below:
(I-1) a polypeptide comprising an amino acid sequence in which a combination of 249th to 251st three amino acid residues is substituted with a combination of three amino acid residues shown in any of (1) to (41) in Table 1 below in an amino acid sequence set forth in SEQ ID NO: 1,
**[Table 1]**
| | | | | | | | | | | | |
|---|---|---|---|---|---|---|---|---|---|---|---|
| (1) | RLF | (8) | ARF | (15) | LTF | (22) | VLF | (29) | CRY | (36) | LFY |
| (2) | RFY | (9) | CVF | (16) | LAF | (23) | VAF | (30) | CLW | (37) | RLW |
| (3) | GGF | (10) | DAF | (17) | MVF | (24) | VCF | (31) | CTY | (38) | RWY |
| (4) | LGF | (11) | GLF | (18) | RVF | (25) | VMF | (32) | FVY | (39) | SFY |
| (5) | VGF | (12) | ISF | (19) | SRF | (26) | VRF | (33) | GSY | (40) | TLY |
| (6) | RGY | (13) | LSF | (20) | TRF | (27) | VEF | (34) | LRW | (41) | VLY |
| (7) | VGL | (14) | LFF | (21) | TLF | (28) | YQF | (35) | LSY | | |
(I-2) a polypeptide in which, in an amino acid sequence in which a combination of 249th to 251st three amino acid residues in the amino acid sequence set forth in SEQ ID NO: 1 is substituted with a combination of three amino acid residues shown in any of (1) to (41) above, one or several amino acid residues other than the combination of three amino acid residues into which the substitution has been introduced are substituted, added, inserted or deleted, and transglutaminase activity at any temperature of 50°C or higher is improved more than transglutaminase activity at the temperature of a polypeptide comprising an amino acid sequence in which the combination of three amino acid residues is not substituted, and
(I-3) a polypeptide in which, in an amino acid sequence in which a combination of 249th to 251st three amino acid residues in the amino acid sequence set forth in SEQ ID NO: 1 is substituted with a combination of three amino acid residues shown in any of (1) to (41) above, sequence identity excluding the combination of three amino acid residues into which the substitution has been introduced with respect to the amino acid sequence set forth in SEQ ID NO: 1 is 70% or more, and transglutaminase activity at any temperature of 50°C or higher is improved more than transglutaminase activity at the temperature of a polypeptide comprising an amino acid sequence in which the combination of three amino acid residues is not substituted.

2. A modified transglutaminase comprising a polypeptide as defined in any of (II-1) to (II-3) below:
(II-1) a polypeptide comprising an amino acid sequence in which a combination of 243rd to 245th three amino acid residues is substituted with a combination of three amino acid residues shown in any of (42) to (60) in Table 2 below in an amino acid sequence set forth in SEQ ID NO: 1,
**[Table 2]**
| | | | | | | | | | | | |
|---|---|---|---|---|---|---|---|---|---|---|---|
| (42) | MPT | (45) | SPS | (48) | RPR | (51) | CGR | (54) | KAL | (57) | MSA |
| (43) | RPT | (46) | LPS | (49) | VPL | (52) | CIR | (55) | LRS | (58) | MSL |
| (44) | SPR | (47) | LPR | (50) | ASS | (53) | ESR | (56) | LGS | (59) | MMR |
| | | | | | | | | | | (60) | QWS |
(II-2) a polypeptide in which, in an amino acid sequence in which a combination of 243rd to 245th three amino acid residues in the amino acid sequence set forth in SEQ ID NO: 1 is substituted with the combination of three amino acid residues shown in any of (42) to (60) above, one or several amino acid residues other than the combination of three amino acid residues into which the substitution has been introduced are substituted, added, inserted or deleted, and transglutaminase activity at any temperature of 50°C or higher is improved more than transglutaminase activity at the temperature of a polypeptide comprising an amino acid sequence in which the combination of three amino acid residues is not substituted, and
(II-3) a polypeptide in which, in an amino acid sequence in which a combination of 243rd to 245th three amino acid residues in the amino acid sequence set forth in SEQ ID NO: 1 is substituted with the combination of three amino acid residues shown in any of (42) to (60) above, sequence identity excluding the combination of three amino acid residues into which the substitution has been introduced with respect to the amino acid sequence set forth in SEQ ID NO: 1 is 70% or more, and transglutaminase activity at any temperature of 50°C or higher is improved more than transglutaminase activity at the temperature of a polypeptide comprising an amino acid sequence in which the combination of three amino acid residues is not substituted.

3. A modified transglutaminase comprising a polypeptide as defined in any of (III-I) to (III-3) below:
(III-1) a polypeptide comprising an amino acid sequence in which a second amino acid residue is substituted with (61) L or (62) F in an amino acid sequence set forth in SEQ ID NO: 1,
(III-2) a polypeptide in which, in the amino acid sequence in which a second amino acid residue in the amino acid sequence set forth in SEQ ID NO: 1 is substituted with an amino acid residue (61) or (62), one or several amino acid residues other than the second amino acid residue are substituted, added, inserted or deleted, and transglutaminase activity at any temperature of 50°C or higher is improved more than transglutaminase activity at the temperature of a polypeptide comprising an amino acid sequence in which the second amino acid residue is not substituted, and
(III-3) a polypeptide in which, in the amino acid sequence in which a second amino acid residue in the amino acid sequence set forth in SEQ ID NO: 1 is substituted with an amino acid residue (61) or (62), sequence identity excluding the second amino acid residue with respect to the amino acid sequence set forth in SEQ ID NO: 1 is 70% or more, and transglutaminase activity at any temperature of 50°C or higher is improved more than transglutaminase activity at the temperature of a polypeptide comprising an amino acid sequence in which the second amino acid residue is not substituted.

4. A DNA encoding the modified transglutaminase according to any one of claims 1 to 3.

5. An expression cassette or a recombinant vector comprising the DNA according to claim 4.

6. A transformant obtained by transforming a host with the expression cassette or recombinant vector according to claim 5.

7. A method for producing a modified transglutaminase, comprising a step of culturing the transformant according to claim 6.

8. An enzyme agent comprising the modified transglutaminase according to any one of claims 1 to 3.

9. A modifier of a protein material, comprising the modified transglutaminase according to any one of claims 1 to 3.

10. A method for producing a modified protein material, comprising a step of allowing the modified transglutaminase according to any one of claims 1 to 3 to act on a protein material.

11. The production method according to claim 10, wherein the protein material is edible.
